# EUROPEAN PATENT APPLICATION

(11) **EP 4 809 982 A1**
(43) Date of publication of application: **23.09.2026**
(21) Application number: 25165112.1
(22) Date of filing: 20.03.2025
(51) Int. Cl.: A61F 2/14

(54) **INJECTOR AND METHOD FOR PREPARING A TISSUE GRAFT FOR DSAEK**

(71) Applicant: TissueGUARD GmbH, 01217 Dresden (DE)
(72) Inventor: TSURKAN, Mikhail, 01217 Dresden (DE)
(74) Representative: Maikowski & Ninnemann Patentanwälte Partnerschaft mbB

(57) **Abstract**

The invention discloses a device, namely an injector (100), for Descement Stripping Automated Endothelial Keratoplasty (DSAEK) comprising a main body, wherein the main body comprises
• a first compartment as transportation and storage section comprising a central axis in longitudinal extension of the main body;
• a second compartment (C1, C2) as insertion section comprising a central axis in longitudinal direction of the main body; and
• a first and a second opening (C4, C3);
wherein the central axis (C5) of the first compartment does not coincide with the central axis (C6) of the second compartment. Further the invention discloses a method for preparing a tissue graft for DSAEK.

## Description

The invention discloses an injector for Descement Stripping Automated Endothelial Keratoplasty (DSAEK) comprising a main body, wherein the main body comprises
- a first compartment as transportation and storage section comprising a central axis in longitudinal extension of the main body;
- a second compartment as insertion section comprising a central axis in longitudinal direction of the main body; and
- a first and a second opening;
wherein the central axis of the first compartment does not coincide with the central axis of the second compartment. Further, the invention discloses a method for preparing a tissue graft for DSAEK.

Ophthalmic tissue transplantation can be significantly enhanced using endothelial keratoplasty ("EK") surgical techniques. These techniques include Descemet Stripping Automated Endothelial Keratoplasty ("DSAEK") or manually done Descemet Stripping Endothelial Keratoplasty ("DSEK"), wherein a layer of the donor cornea comprising the Descemet's membrane and attached endothelial cells, along with a thin layer of donor stromal tissue, is transplanted into a recipient's eye. DSAEK is produced with the help of a specific instrument, but there is no principal difference between tissue produce as DSEK and DSAEK and their behaviors are identical. Descemet Stripping Automated Endothelial Keratoplasty (DSAEK) is the second most commonly performed endothelial keratoplasty in the EU and USA, while it remains the leading procedure worldwide.

Descemet's Stripping Automated Endothelial Keratoplasty (DSAEK) is a minimally invasive, small-incision surgical technique that has become the leading advancement in posterior lamellar keratoplasty procedures, commonly associated with the broader term "endothelial keratoplasty." The DSAEK procedure entails the removal of diseased Descemet's membrane and endothelial cells through a minor corneal incision, followed by the transplantation of a posterior lamellar donor corneal lenticule. This donor tissue is prepared using the Automated Lamellar Therapeutic Keratoplasty (ALTK) unit (Price M O, Price F W Jr.: "Descemet's stripping with endothelial keratoplasty: comparative outcomes with microkeratome-dissected and manually dissected donor tissue"; Ophthalmology; 2006 November; 113(11):1936-42).

A major technical challenge of DSAEK is the introduction of the donor lenticule into the anterior chamber (AC) through a small incision while minimizing endothelial damage. The conventional method, commonly referred to as the 'taco insertion' technique, requires folding the donor lenticule and inserting it through a 5 mm corneal or scleral incision, using non-compressing forceps to grasp the folded tissue. However, this approach has been criticized for its potential to cause significant endothelial cell damage. Reported rates of primary graft failure due to intraoperative endothelial cell loss vary from 6% to 45%, depending on the study and technique used (Mearza A A, Qureshi M A, Rostron C K: "Experience and 12-month results of Descemet-stripping endothelial keratoplasty (DSEK) with a small-incision technique"; Cornea 2007 April; 26(3):279-283).

Endothelial damage in DSAEK primarily results from the mechanical folding of the donor tissue, the compression exerted by the holding forceps, and intraocular manipulations required to unfold the Graft within the AC in the absence of an ophthalmic visco-surgical device (OVD). Recent laboratory models of DSAEK have confirmed that the greatest endothelial cell loss occurs during these steps-when the donor lenticule is folded for insertion and later unfolded inside the anterior chamber (Lee W B, Sy H M, Holley G P, Edelhauser H F: "Descemet's Stripping Automated Endothelial Keratoplasty (DSAEK): IntraOperative Effects on the Donor Corneal Endothelium"; IOVS supplement; 2007; abstract 1131). The risk of endothelial cell damage is even more pronounced when the anterior chamber becomes shallow during the procedure.

Despite the use of commercially available non-compression forceps (Goosey forceps, model no. 19090, Moria, Antony, France), significant endothelial damage still occurs with the conventional folding technique. This damage is primarily attributed to direct contact between folded endothelial surfaces at the point where the folding forceps are applied, as well as along the folding crease (Mehta J S, Por Y M, Beuerman R W, Tan D T: "Glide Insertion Technique of Donor Cornea Lenticule during Descemet's Stripping Automated Endothelial Keratoplasty"; J Cat Refract Surg; in press). Recent findings indicate that the mean endothelial cell loss using this approach is approximately 39%, further highlighting the limitations of the folding technique because such damage limits the graft feasibility. This is true for any DSAEK graft above 60 microns.

Corneal transplantation is one of the most common transplant procedures worldwide, with DSAEK representing approximately 30% of all corneal transplants. DSAEK offers advantages such as reliable handling during transplantation due to the relative thickness of the Graft, which is typically between 100 to 200 microns, and reduced surgical complexity compared to Descemet's Membrane Endothelial Keratoplasty ("DMEK"). The modern techniques allow the DSAEK creation around and slightly under 100 microns which is the most common technique. Recently, DSAEK tissue could be created with thickness even under 50-60 microns, also called nanoDSAEK or microDSAEK or ultrathinDSAEK. Thinner DSAEK graft decreased the surgical complications but issues with folding remained. It was revealed that the thin DSAEK graft can be partially folded and delivered in a short term, but c it should not be done for more than around two day (Cornea 43(1):p 111-119, January 2024. | DOl: 10.1097/ICO.0000000000003385). It was found that thinner compartment could be more beneficial for ultrathin DSAEK below 60 microns, I hypostatized that different devices should be used for DSAEK graft of different thickness

Despite the advantages of DSAEK, challenges remain in the storage, transport, and delivery of donor tissue. Delivery of ophthalmic tissue, including the donor stromal and endothelial layers, requires careful handling to prevent damage. The donor tissue must be meticulously removed from the donor cornea, preserved in a sterile environment, transported to the surgical site, and inserted into the recipient's anterior chamber. This preparation of the graft is commonly made at an Eye bank or specified hospital facility. Next the prepared graft has to be transferred to the surgical theater for following transportation by a surgeon. Originally, the devices for graft transplantation and devices for surgical implantation of DSAEK were different. The graft after preparation, could be put back on the cornea and delivered on the cornea in its original container. Alternatively, it could be put in a container with a medium and transported this way. In any way, the surgeon has to manipulate the graft in a surgery theater to remove it from the container and put it in a device used for transplantation.

Ensuring that the endothelial layer remains undamaged during transport and implantation remains critical for efficiency, efficacy and lifetime of the implanting graft which together influence strongly on the patient quality of life. The corneal endothelium plays a vital role in maintaining corneal transparency by actively pumping fluid out of the corneal stroma, preventing the development of edematous haze and cloudy vision. As the corneal endothelium does not regenerate after damage, preserving the integrity of this layer is essential for successful transplantation.

The most advanced use of the DSAEK devices is when the tissue is delivered to the surgery already inside of the device which makes it safer for the transport and transplantation. This method is named "preloaded DSAEK" and was introduced a few years ago. For DSAEK procedures, a preloaded delivery device is especially advantageous as it minimizes the time and complexity of the surgical procedure. By delivering the graft in an "endo-in" configuration, where the endothelial cells face inward toward the recipient's anterior chamber, the device simplifies the unfolding and positioning process. The additional thickness and support of DSAEK grafts reduce the risk of unintended folding or damage during insertion, ensuring reliable and consistent outcomes.

The growing demand for preloaded DSAEK solutions requires a device that enables seamless integration from preparation to implantation. Conventional DSAEK injectors, including glass cannulas and plastic devices, exhibit several limitations:
1. High fragility (e.g., borosilicate glass injectors).
2. Inability to validate tissue quality microscopically within the injector.
3. Limited control over graft orientation during implantation.
4. Complex multi-component designs that complicate handling and increase costs.

Moreover, the graft can be prepared in various thickness, which is typically between 30 to 200 microns. The current devices are not optimized for such a broad range or thickness of DSAEK grafts. Mostly because the change in the graft behaviour and handling depends on its thickness. Very thin DSAEK grafts (below 60 micrometres) are closer in its properties to Pre-Descemet's endothelial keratoplasty PDEK or in some extend DMEK grafts while thick DSAEK grafts (>60 microns) are close in its mechanical properties to cornea stroma and should not be compressed for long time due to possible damage including endothelial damage. The DSAEK graft preparation are very varies between eye banks technicians and surgeons. Currently all types of DSAEK graft from 30 to 200 microns are still widely used and require specific devices for them. It seems that different devices are required for DSAEK with different thickness, due to significant difference in the properties of DSAEK grafts. In conclusion, current devices do not fully address the needs of eye banks, surgeons, or patients.

The first-generation devices designed for DSAEK, such as metal spatulas like the Busin Glide, present several significant drawbacks. The primary disadvantage is the large incision size required (approximately 5.0-5.5 mm), which leads to increased astigmatism and prolonged healing compared to smaller-incision systems. Additionally, the Busin Glide can only be used with the pull-through technique, which necessitates the use of additional instruments, such as ophthalmic microforceps. The metal construction of the device also poses challenges in visualizing the delicate donor tissue and contributes to corneal endothelial cell loss due to mechanical stress during surgery, which remains a major clinical concern.

These limitations-including large incision size, reduced visibility, the need for specific instruments, and the risk of endothelial cell damage-highlight the need for innovation in DSAEK instrumentation. As a result, metal spatulas like the Busin Glide can no longer be considered competitive with modern DSAEK devices.

Currently available devices for DSAEK include fragile, multi-component cannulas made of borosilicate glass, such as the transplantREADY DSAEK Weiss Glass Cannula or Weiss Glass Cannulas- Miracles in Sight and Besson Tube and plastic devices with complex geometries, such as the EndoSerter and EndoGlide.

However, these devices present several challenges:
- Glass injectors (e.g., transplantREADY): Extremely fragile due to thin walls, making them prone to breakage. Their round structure complicates microscopic evaluation of the tissue,
- Plastic injectors: Also have round geometries and do not have glass like transparency that hinder tissue evaluation before transplantation. Moreover, their multi-component design makes them difficult to handle and increases costs.

These devices can be adapted for preloaded DSAEK, but they require special containers and adapters, making them uneasy and expensive to use. These challenges highlight the need for an innovative solution in preloaded DSAEK technology.

The most common transplantation technique for DSAEK is pull through if a cornea surgent pulls the graft from the carrier device into patient eye with forceps. Using forceps always creates damage for the graft. Damage to the endothelial cells during this process can compromise the success of the transplant and lead to suboptimal visual outcomes.

Unlike DMEK grafts, which tend to scroll tightly with endothelial cells exposed (the "endo-out" configuration), DSAEK grafts do not scroll due to their greater thickness and presence of stromal support. The natural conformation of DSAEK is flat with a slightly curved surface that resembles the cornea surface. Therefore, this conformation should be maximally retained in order to prevent the endothelial cell loss (ECL) associated with preloaded DSAEK graft storage transportation and transplantation using carriers. In contrast, the device should be transplanted from a smallest incision cut to minimize the patient's eye damage and possible complications. Ophthalmic tissue storage and delivery devices must address these challenges, enabling surgeons to safely handle and implant the Graft while minimizing endothelial cell loss. In other words, they should be easy to use. It is difficult for current devices to provide all of these requirements.

Commonly, carriers with larger interior compartments, allowed grafts to spread more evenly, minimizing folds and overlap, which reduced ECL while narrow carriers caused greater compression, folding, and overlap of grafts, leading to higher ECL The dimension of the DSAEK grafts are very strongly depending on the infrastructure and the preparation techniques as well as the skill of the personal. Ultra-thin DSAEK or also named nano-thin DSAEK are closer to 40-micron thickness and have mechanical behaviour that defines their conformation closer to Pre-Descemet's endothelial keratoplasty (PDEK) grafts. PDEK is a kind of endothelial keratoplasty, where the pre descemet's layer (PDL) along with descemet's membrane (DM) and endothelium and slightly roll at the ends. This DSAEK tissue can be partially rolled for some time, which is very unwanted for larger DSARK tissues.

Several patents have been filed for devices designed to facilitate the delivery of donor endothelial grafts into the anterior chamber during corneal surgeries, such as Descemet's Membrane Endothelial Keratoplasty (DMEK) and Descemet Stripping Automated Endothelial Keratoplasty (DSAEK).

US 2004 0039401 A1 discloses a device delivering implants with liquid flow. It does not have a suitable implantation part and is designed for small retina implants, which is why the device would damage DSAEK grafts which are much larger. The insertion design is easy to hold the tissue but creates a large incision cut - which is very unwanted in DSAEK surgeries. Other recent innovation included NS Endo-Inserter: PCT/JP2011/067665 and PCT/JP2015/055624.

These techniques were where developed to reduce endothelial cell damage during DSAEK. The NS Endo-Inserter allows the graft to flow into the anterior chamber in a balanced salt solution, lessening intraoperative mechanical stress on the graft tissue. The device has a circular or oval cross-section and requires specific devices and connectors for implantation. It is a variation of the Endoglide cartridge which was described in detail above and has the same disadvantages. Another patent describes the use of Endoserter for preloaded DSAEK (US12070414B2), but this device requires a pull-through technique, and as described above and has the same disadvantages.

It was the purpose of the invention to overcome the disadvantages of the state of the art and provide an injector which is optimized for DSAEK tissue grafts.

### Detailed Description

The invention provides a device, namely an injector, optimized for Descemet's Stripping Automated Endothelial Keratoplasty (DSAEK), designed to facilitate secure support, storage, transport, and precise microscopic evaluation of a tissue graft. In the following the device is also referred to as an injector. Further, the injector can also be used for the implantation (introduction) into a living body. The injector is basically a kind of cannula or cartridge consisting of distinct sections.

The invention provides a device for Descement Stripping Automated Endothelial Keratoplasty (DSAEK) comprising a main body, wherein the main body comprises
- a first compartment as transportation and storage section comprising a central axis in longitudinal extension of the main body;
- a second compartment as insertion section comprising a central axis in longitudinal direction of the main body; and
- a first and a second opening;

According to the invention the main body comprises a first and a second compartment which are to be understood as two distinct parts of the device. The first opening is arranged on the first compartment of the main body. The second compartment follows seamlessly on the first compartment and the second opening is arranged on the second compartment of the main body. The device is a hollow device, wherein the first and the second opening form the openings to the interior of the main body.

The first compartment is used as transportation section, which means that the DSAEK tissue grafts can be stored and transported in this section.

The first compartment defines the level of tissue graft compression due to its shape. In one embodiment the first compartment has a round, rectangular or squared shaped part. In one embodiment the first compartment has a rounded, squared, or rectangular shape or a squared or rectangular shape with rounded corners. Advantageously, the shape of the first compartment can be adjusted to accommodate tissue grafts with varying thickness.

Advantageously, the first compartment can be tailored for all types of DSAEK tissue grafts depending on their storage and transportation requirements in terms of time and medium characteristics (swelling or none swelling medium). Different shapes of the first compartment are used to optimize storage and transportation conditions for different types of DSAEK grafts. Accordingly, the first compartment is designed to accommodate a DSAEK graft securely, preventing unnecessary movement or compression that could damage endothelial cells.

In one embodiment the first compartment is a squared rectangular with or without rounded corners. Opposite walls of the first compartment are parallel to each other which simplifies of the injection of the tissue graft. The tissue graft is compressed in this embodiment therefore this design can be applied only for a short transplantation or preparation in surgical theater. This modification can be used freely for an ultra or nano thin DSAEK tissues under 50 microns.

In one embodiment the first compartment has a rounded shape, which decreases the compression of the tissue graft. Preferably the second compartment has straight walls which makes it easy for the tissue graft to pass during an injection. This design is suitable for a longer-term transportation and a short storage. This modification is suitable for all types of the DSAEK tissues including the thickness of up to 150 microns.

In a further embodiment the first compartment has a rectangular shape with rounded corners. The first compartment is more elongated compared to the embodiment described before. The first compartment is designed to accommodate a DSAEK tissue graft in its natural state. Which means in the shape of a slightly curved flat circle with a diameter of less than 9,5 mm. Accordingly, the dimensions of the first compartment are such that a DSAEK tissue graft in its natural state perfectly fits inside of the first compartment. The shape of the first compartment is such that in the hollow inner part a circle with a diameter between 9,5 mm and 6 mm created. This design enables the storage and transportation of DSAEK tissue grafts of any size, including full-thickness stromal grafts, while preventing unnecessary mechanical compression. It also allows for long-term storage and safe transport of the tissue graft within the injector, minimizing mechanical trauma. The first compartment provides due to its size a large medium volume for the tissue graft.

The shapes of the first compartment define the compression of the DSAEK tissue graft and optimized for DSAEK tissue grafts of different dimension in term of storage and transportation, in which one embodiment is suitable for a very short shortage, transportation and preferably very thin DSAEK tissue grafts under 60 microns; one embodiment is suitable for common storage and transportation of few days for DSAEK tissue grafts under 100 microns; and a further embodiment allows for long term storage, transportation and up to full stroma thickness tissue grafts because it keep the tissue graft in its native confirmation.

The second compartment is used as insertion section, which means the DSAEK tissue graft is positioned in this compartment before transplantation. The second compartment is between the first compartment and the second opening.

There is a transition area between the first and the second compartment which connects the geometry of both compartments.

In one embodiment the second compartment comprises two opposing angled surfaces, wherein the angle is between 0° and 10°, preferably between 0° and 5°. The angle is measured between the surface and the straight line perpendicular to the vertical line. Therefore, the surfaces are slightly angled towards the second opening of the device.

Accordingly, in one embodiment opposing walls of the second compartment are flat and parallel, which means the angle is 0°.

If opposing walls are parallel or include a small angel (0-10° towards the small opening of the device) to each other facilitates smooth passage of the tissue graft. This configuration enhances surface tension forces, facilitating the smooth passage of the tissue graft through the whole second compartment up to the second opening, where the tissue graft experiences maximum compression. By minimizing the contact area between the rolled tissue graft and the flat surfaces of the second compartment and in one embodiment also the first compartment, the design ensures a smoother tissue graft transition. In contrast, round or oval geometries create a larger surface interaction between the injector and the tissue graft due to their closer shape conformity, which increases the risk of tissue graft damage.

In one embodiment the injector has opposing flat surfaces in the first compartment and/or in the second compartment.

Each first compartment can be designed to provide optimal tissue graft protection while ensuring secure handling. The varying geometries regulate compression levels, reducing unnecessary mechanical stress and potential cell damage.

According to the invention the central axis of the first compartment does not coincide with the central axis of the second compartment. The central axis is defined in longitudinal direction of the main body and therefore of the whole injector. The first component and the second component are designed in asymmetry as discussed in more detail in the following. This asymmetry should be small enough for a microforseps to be able to frilly pass through the hole device from the second opening to the first opening in order to grab and pull DSAEK graft inside of the device. In one preferred embodiment the central axis of the first compartment is shifted parallel to the central axis of the second compartment.

The asymmetry is designed in the way that the central axis of the second compartment is shifted from the central axis of the first compartment up to the diameter of the second opening. Preferably the central axis of the second compartment is shifted from the central axis of the first compartment between 1/3 to 1/2 of the opening diameter of the second opening.

DSAEK tissue graft is a naturally flat with a small curve tissue and the best is to be stored in such conformation. Unfortunately, the DSAEK tissue graft rolls when passing through injector devices. The injector according to the invention incorporates engineering features that allow precise control of DSAEK tissue graft conformation before and during injection. If the second compartment has flat surfaces passing the tissue graft with a flow is simplified because surface tension in the flat surfaces minimizes the contact between the tissue graft and the device especially for hydrophobic surfaces. The second compartment is positioned off the central axis or asymmetrically relative to the first compartment. This asymmetrical design significantly increases the likelihood of the tissue graft forming a single scroll, which applies less compression on the tissue, reducing endothelial cell loss (ECL). By promoting a single scroll formation, this design minimizes mechanical stress on the tissue graft, preserving endothelial integrity.

In a further embodiment the second compartment comprises a grid positioned after the transition area from the first compartment, which can induce a double scroll formation. This configuration aligns with the preferences of some corneal surgeons who favour this technique for controlled tissue delivery.

One of the key features of the inventive injector is the asymmetrical shift of the central axis of the second compartment, positioning it to the side relative to the central axis of the first compartment. This asymmetry induces controlled rolling of the DSAEK tissue graft as it transitions from first compartment to the second compartment during injection or retrieval, ensuring the formation of a single roll. This configuration is considered optimal.

Accordingly, the second component being off-axis, promotes a single-roll formation of the DSAEK graft, enhancing positioning upon implantation. Further, the second compartment maintains the correct orientation of the tissue graft, reducing the risk of misalignment or loss during surgical procedures.

In one embodiment of the invention, the inner hollow of the compartments (main body, taper area and second opening) of the device is the same as the outer shape of these compartments of the device. In another embodiment, the shape of the inner hollow compartments of the taper area and second opening of the device can be different from the outer shape of these compartments of the device. This has the advantage, that the best performance for both stable orientation and feasible microscopic investigation of the tissue inside the device as well as optimal functioning during the operation process (such as wound closure of the surgical incision while the device is inserted) is ensured.

According to the invention, the first compartment has a constant wall thickness. The wall thickness is between 0.1 mm and 1.0 mm, preferably between 0.1 mm and 0.8 mm, more preferably between 0.1 mm and 0.6 mm or between 0.2 mm and 0.4 mm.

The wall thickness of the second compartment and the second opening can vary and depends on the overall shape and/or the difference between the shape of the outer and inner wall of the second compartment and the second opening. In case the second opening has the same shape as the second compartment, the wall thickness is constant. In this event, the wall thickness is 0.1 mm and 1.0 mm, preferably between 0.1 mm and 0.8 mm, more preferably between 0.1 mm and 0.6 mm or between 0.2 mm and 0.4 mm. Most preferably, the wall thickness is 0.25 mm.

In a further embodiment, the injector has a total length between 20 mm and 100 mm, preferably between 20 mm and 50 mm, more preferably between 25 mm and 35 mm.

In one embodiment the first compartment and/or second compartment is transparent or translucent. Preferably, the first and second compartment are transparent or translucent. The transparent, preferably rounded rectangular design allows for detailed microscopic inspection of the tissue graft while securing it in place during delivery and implantation.

In one embodiment the injector consists of glass or plastic. In a preferred embodiment the injector is made from polymeric translucent material preferably fully transparent polymeric materials.

In one embodiment of the invention, the device is made from glass, preferably borate glass.

In a more preferred embodiment of the invention, the device is made of transparent plastics which are tougher than glass but show similar transparency. This type of glass is in the following called "glass-like plastic". Moreover, plastics can be produced with much thinner wall thickness than any glass (e.g. via injection molding or 3D printing) without the risk of breakage during the operation procedure, opening new possibilities for even smaller incisions on the patient's eye than possible with conventional devices. The transparency of the device enables the application of evaluation and quality control of the tissue implant or graft by precision microscopy methods such as specular microscopy, slit-lamp examination, light microscopy and optical coherence tomography. In a most preferred embodiment, the plastics used for preparing the device according to the invention is highly transparent. According to the invention, the refractive index (rᵢ) is in the range of rᵢ = 1.30 to rᵢ = 1.71, preferably rᵢ = 1.30 to rᵢ = 1.65, most preferably 1.30 to rᵢ = 1.60, which is similar to the rᵢ of balanced salt solution (BSS, around 1.33 to 1.34 at 20 °C and around 600 nm), which can be used for the loading, evaluation and/or storage of tissue transplants within the device. The term 'BSS' comprises buffered aqueous saline solutions such as Hank's BSS, Earle's BSS, Tyrode's salt solution, Alsever's salt solution, phosphate-buffered saline (PBS), Tris-buffered saline (TBS), Puck's salt solution, Gey's salt solution, Ringer's salt solution, Simm's salt solution and related buffered saline solutions. It is also possible to use plastic compositions, such as combinations, mixtures, blends or copolymers of two or more plastics to manufacture the device of the invention. Generally all plastic compositions which are translucent more preferably transparent are suitable for the device of the present invention because they are fitting under these requirements. In a preferred embodiment, the device is made of polyacrylates, polycarbonates or polystyrenes.

In one embodiment of the present invention, the inner surface of the injector comprises a hydrophobic coating.

Hydrophobic coatings smooth the surface and create a strong surface tension, preventing the implant to "touch" the surface while being in solution. Hydrophobic coatings suitable for the injector of the present invention are listed in Table 1. In one embodiment of the present invention, the inner surface of the injector comprises a hydrophobic coating, coating blends, coating mixtures or other combinations of hydrophobic coatings selected from Table 1.

In a preferred embodiment of the present invention, the inner surface of the device comprises a hydrophobic coating, selected from coatings comprising acrylate, organo-siloxane, silane, epoxy, a polymer, Molybdenum disulfide, Molybdenum disulfide/graphite, Tungsten disulfide or graphite, preferably selected from coatings comprising a polymer, organo-siloxane, silane, acrylate or epoxy.

In one further embodiment the outer wall of the second component is aligned with the outer wall of the first opening in the way that it can be produced by injection molding. Preferably, in one embodiment the wall angel or curvature towards the inside of the injector is no less than 1 percent.

The first opening is positioned on the first compartment. In one embodiment the first opening comprises a luer connector, which allows a good or secure attachment of the device to various syringes as well as safe loading the DSAEK graft into the device.

The first opening serves in this embodiment as a luer connector which can have different diameters, enabling secure attachment to various syringes or tubing. Further it can be a Luer Slip or Luer Lock and alternatives for the secure connection to a syringe.

The second opening is positioned on the second compartment. The second compartment and the second opening are used to deliver the tissue implant into the eye. The second compartment acts as the final passage for the tissue graft before implantation. The second opening functions like a nasal for ejecting the tissue graft into the eye. The second compartment and therefore the second opening are shifted from the central axis of the first compartment so when DSAEK tissue graft passes the second opening, it enhances a single roll formation of the tissue graft. The off-axis design ensures that the tissue rolls in a controlled manner, preventing undesired folding or distortion of the tissue graft during insertion.

In one embodiment the second opening is located on the second compartment and has a diameter between 1.0 mm and 1.8 mm, preferably between 1.3 and 1.5 mm. This allows for a DSAEK tissue graft to be implanted into the eye with a minimum incision cut under 3,5 mm with a minimum cell lost derange.

The second opening can be open during storage and transportation for medium diffusion to and out the device. The geometry of the injector allows for the insertion of the tissue graft by flow of a liquid without pull through technique. The invention thereby ensures minimal cell loss, easy handling, and a controlled environment for the DSAEK tissue graft throughout its handing, storage, transplantation and implantation.

In one embodiment the second opening may be elliptical, circular, biconvex, or a rounded rectangle, ensuring a configuration that is small enough to fit through a minimal surgical incision while maintaining optimal positioning of the tissue graft or implant. The optimal connection is from 1.0 till 1.8 mm in internal diameter with the best for DSAEK tissue is between 1.3 and 1.5 providing the best compromise between the size of a minimal surgical incision and a minimum cell damage due to the DSAEK tissue passing.

In a further embodiment the device comprises a cap or a lid for the first and/or for the second opening.

Using the cap or the lid the first opening can be secure closed which prevent the tissue graft from escaping during the storage and transportation in a container.

In one embodiment a cap or lid can be securely attached to the first opening to prevent tissue graft escape during storage and transportation, while the second opening can be left open to allow medium diffusion, maintaining an optimal hydration level of the tissue graft.

In one embodiment a cap or lid can be used to close secure from both ends, the second opening can be closed tightly with a soft cap preferably out of silicon or rubber. A secure cup may be installed closing with a thread, then a thread is made on the device no less than 0.5 cm from the second opening.

In one embodiment the central axis of the first compartment is not shifted to the central axis of the second compartment. In this embodiment the device has a symmetrical configuration. All further features described apply for this embodiment as well. Advantageously, still the device allows a DSAEK graft to be stored in its native conformation inside the device.

Further, the invention provides a method for preparing a tissue graft for DSAEK using the device according to the invention comprising the steps
- loading a DSAEK tissue graft into the first compartment;
- arranging a macroporous material in the second opening;
- positioning the tissue graft within the second compartment using a liquid flow.

All features described in connection with the injector apply for the method for preparing a tissue graft for DSAEK as well and vice versa.

Additionally, the invention includes a method for preparing tissue graft for an implantation. In the current state of the art, tissue must be rinsed with Balanced Salt Solution (BSS) or a similar buffer and injected with it. If a pull-through technique is used, the shape of the device is not critical since tissue graft movement is controlled by microforceps pulling the tissue graft. However, this technique is associated with additional tissue damage and potential harm to the recipient's eye. The tissue graft can be pushed from the large opening toward the tip opening with a plunger for implantation. While this allows for precise control of the injection, the pushing forces significantly compress the tissue graft from one side, causing damage. The gentlest and therefore preferable approach is to push the tissue graft through a device using liquid flow. However, when surgeons use liquid injection, the speed at which the tissue graft moves through the device depends on its shape and varies across different sections. Controlling the injection rate during surgery is critical to preventing the tissue graft from moving too quickly or "shooting" into the eye, which requires significant attention and skill from the surgeon. Closing the small insertion with a porous material preferably macroporous material or a washing device, as described in WO 2020/169740 A1, during the injection process ensures both efficiency and security. The liquid passes smoothly through the macroporous material, while the Graft remains securely positioned at the device's tip, unable to penetrate the material. This type of material has a minimal contact area on its subphase, preventing cell damage to the tissue graft. Additionally, during the washing process, the tissue graft touches the material only with its thin side, which has minimal cell presence, thus preserving the overall cell count.

Accordingly, the method of the present invention uses a liquid flow to transport the tissue graft through the injector while a macroporous material closes the second opening. Suitable macroporous materials are known to the person skilled in the art and are described in WO 2020/169740 A1.

The method according to the invention significantly simplifies and accelerates the washing and implantation procedure while ensuring the tissue graft remains completely secure.

Furthermore, the invention provides a method for implanting a tissue graft for DSAEK using the device according to the invention comprising the steps
- loading a DSAEK tissue graft into the first compartment;
- arranging a macroporous material in the second opening;
- positioning the tissue graft within the second compartment using a liquid flow;
- remove the macroporous material from the second opening;
- eject the tissue graft from the injector into the anterior chamber of an eye.

All features described in connection with the injector and/or the method for preparing a tissue graft for DSAEK apply for the method for implanting a tissue graft for DSAEK as well and vice versa.

According to the method for implanting the tissue graft which is positioned inside the second compartment according to the method for preparing a tissue graft for DSAEK of the invention is ejected into the eye through a liquid flow after removing the macroporous material from the second opening. Once the tissue graft reaches the second opening positioning area, its injection becomes very simple, requiring minimal liquid movement-similar to the process used for DMEK tissue.

Further a method for storage or transporting a tissue graft for DSAEK using the device according to the invention in a container with medium comprising the steps
- filling the device with medium;
- loading a DSAEK tissue graft into the first compartment;
- closing the large opening with a cap preferably a luer lock lid;
- positioning the device into a container with medium, for transportation;
is provided.

Further a method for storage or transporting a tissue graft for DSAEK using the device according to the invention in a container without medium comprising the steps
- Filling the device with medium;
- loading a DSAEK tissue graft into the first compartment;
- closing the large opening with a cap preferably luer lock lid;
- closing the smaller opening with a cap
- positioning the device into a container, for storage and transportation.
is provided.

In one embodiment the method further comprises the step of transporting the device (e.g. shipping), disinfect the device and remove the cap for transplantation.

All features described in connection with the device according to the invention apply for all methods of the invention as well and vice versa.

Suitable mediums to be used in such devices are well known to the person skilled in the art.

The present invention relates to an optimized device specifically designed for Descemet's Stripping Automated Endothelial Keratoplasty (DSAEK) tissue transplantation. The device is engineered to facilitate the secure support, storage, transportation, and precise microscopic evaluation of a tissue graft or implant during processing, as well as its introduction into a living body.

The invention introduces several groundbreaking features:
1. The segmented design allows easy adaptation for all types of DSAEK Tissues, by slightly changing the shape of the device's storage and transportation compartment, namely the first compartment.
2. The first compartment ensures safe containment and preservation of the tissue graft or implant during handling, storage, and transportation.
3. Flat Surface Geometry of second compartment: Enhances microscopic validation of grafts prior to implantation, ensuring tissue quality. Facilitating controlled delivery and accurate placement of the tissue into the recipient site.
4. Hydrophobic, Transparent Plastic Construction: Prevents tissue adhesion and facilitates visual inspection.
5. Asymmetrical Design: Ensures controlled graft orientation during both loading and implantation, suitable for both pull-through and injection techniques. Enhance efficient single-roll formation for DSAEK and damage-minimized tissue graft insertion.
6. Luer Connector: Allows secure attachment of caps, preventing tissue graft escape during transport or handling.
7. Compact Surgical Wound: Requires less than 3.5 mm incision, reducing postoperative complications.
8. Preloaded DSAEK: To facilitate transplantation the device can be preloaded with the DSAEK donor tissue. This device ensures precise delivery of the tissue graft into the recipient's anterior chamber, reducing the risk of damage caused by manual handling.

Further, the invention provides several key advantages over conventional DSAEK tissue implantation methods:
1. Minimally Invasive
   o The diameter dimension (1.0-1.8 mm) of the second opening allows implantation through an incision less than 3.5 mm, reducing surgical trauma and endothelial cell loss.
2. Optimized Graft Compression
   ∘ The shape of the first compartment controls graft compression, ensuring compatibility with varying thicknesses of DSAEK tissue and time of storage and transportation from local delivery of very thin DSAEK to national delivery of DSAEK tissue and international log term delivery and storage.
   o The design allows the tissue graft to transition from a rounded or squared shape of the first compartment to a fully flat configuration in the second compartment, simplifying the passage of the tissue graft.
3. Secure Storage and Transportation
   ∘ The different designs provide solutions for short-term, medium-term, and long-term storage.
   ∘ The device can be sealed or closed with a cap to prevent accidental loss or contamination of the tissue graft during transport.
4. Non-Traumatic Graft Delivery
   ∘ The geometry allows graft insertion via liquid flow, eliminating the need for a pull-through technique that could damage endothelial cells.
   ∘ The off-center insertion channel promotes a single-roll formation, ensuring correct positioning of the tissue graft inside the eye.
5. Versatile Compatibility
   ∘ The luer connector enables secure attachment to various syringes and adapters for easy graft loading and injection.
   ∘ The injector can accommodate grafts of different sizes and thicknesses, making it suitable for various patient needs and surgical preferences.
6. Steril delivery
   o If closed from both ends with sterile cups, the DSAEK implant may be stored, transported and delivered to an eye using hydraulic fluid in the device itself without the need of a separate container. The surgeon can disinfect the device by whipping and remove the cap from the device for transplantation.

The device is specifically designed for DSAEK corneal transplantation, a procedure where the diseased Descemet's membrane and endothelium are replaced with a healthy donor graft. This device ensures:
- Efficient graft preparation and loading
- Secure transport without endothelial damage
- Minimized incision size and reduced endothelial cell loss
- Washing with macroporous materials with securing the graft at the tip of the device.
- Controlled graft positioning and roll formation during insertion

This novel device presents a significant advancement in DSAEK tissue transplantation by addressing key challenges related to storage, transportation, and implantation. Its precise design optimizes graft handling, ensuring that endothelial cell loss is minimized while making the surgical process more efficient and less invasive. The versatility of different designs of the first compartment ensures suitability for various clinical scenarios, from short-term to long-term storage and from thin grafts to full-thickness grafts. The unique injection and insertion features allow seamless integration with existing surgical techniques, making this device a valuable tool for corneal surgeons. This design enhances surgical precision, minimizes trauma to endothelial cells, and ensures optimal graft viability, making it a significant advancement in DSAEK transplantation techniques.

None of the devices with movable parts can be compared with the present invention invention because the pushing or pulling DSAEK graft has shown its complexity. The injector according to the invention uses the best delivery method, with only liquid flow and no mechanical parts moving within the device. The device is optimized in its dimension to DSAEK graft, and therefore, a variety of delivery devices for DMEK and Retina graft delivery cannot be compared as they have been shown to have poor feasibility for DSAEK graft implantation.

In the following the invention is further described by 1 table, wherein

**Table 1: shows hydrophobic coatings for the inner compartment of the device, where any listed coatings, coating blends, coating mixtures, or other combinations of the listed coatings can be used.**

| **Table 1** | |
|---|---|
| Coatings comprising Acrylate: e.g. Glazon^{®} | |
| Coatings comprising Organo-siloxane: e.g. SILGLIDE^{®} | |
| Coatings comprising Silane: e.g. Siliclad^{®}, Glassclad^{®}, Chemlon^{®} | |
| Coatings comprising Epoxy: e.g. Everslik^{®}, Castall^{®} | |
| Coatings comprising a hydrophobic polymer | |
| | • Poly(tetrafluorethylene) (PTFE): e.g. Teflon^{™}, Teflon^{™} -S, Teflon^{™}-PFA, Teflon^{™}-TFE, Teflon^{™}-FEP, SilverStone^{®}, SilverStone^{®} Supra, Xylan^{®}, Vydax^{®}, Everlube^{®} 72-series, Sandstrom^{®} Poxylube^{®}, Emralon^{®}, Excalibur^{®}, Ultralon^{®}, LubriSkin^{™}, DuraSkin^{™}, ShieldSkin^{™}, HardSkin^{™}, DuraBond^{™} |
| | • Perfluorpolyether (PFPE): e.g. Krytox^{®} |
| | • Poly(vinylidene fluoride) (PVDF): e.g. Dykor^{®}, Kynar^{®}, Solef^{®} |
| | • Poly(*para*-xylylene) (Parylene): e.g. Parylene AF-4, Parylene C, Parylene C-UVF^{®}, Parylene D, Parylene HT^{®}, Parylene N, Parylene X, ParyFree^{®}, microRESIST^{™} |
| | • Poly(*co*-ethylene-chlorotrifluoroethylene) (ECTFE): e.g. Halar^{®} |
| | • Poly(co-tetrafluoroethylene-hexafluoropropylene) (FEP): e.g. Precision Coating^{®} PC 9020 |
| Coatings comprising Molybdenum disulfide (MoS₂): e.g. Ecoalube^{®}, Perma-Slik^{®}, Esnalube^{™}, Lube-Lok^{®}, Everlube^{®} 73-series, Sandstrom^{®} #099, Electrofilm^{®}, Molykote^{®}, Gun-Kote^{®} | |
| Coatings comprising MoS₂/Graphite: e.g. Lube-Lok^{®}, Kal-Gard^{®} | |
| Coatings comprising Tungsten disulfide (WS₂): e.g. Perma-Slik^{®} RWAC | |
| Coatings comprising Graphite: e.g. Sandstrom^{®} #75C | |

In the following the invention is further described by 5 figures.
- Figure 1: (A) to (C) illustrate one embodiment of the injector according to the invention;
- Figure 2: (A) to (C) illustrate one embodiment of the injector according to the invention;
- Figure 3: (A) to (C) illustrate one embodiment of the injector according to the invention;
- Figure 4: (A) to (C) illustrate one embodiment of the injector according to the invention;
- Figure 5: (A) to (C) illustrate one embodiment of the injector according to the invention.

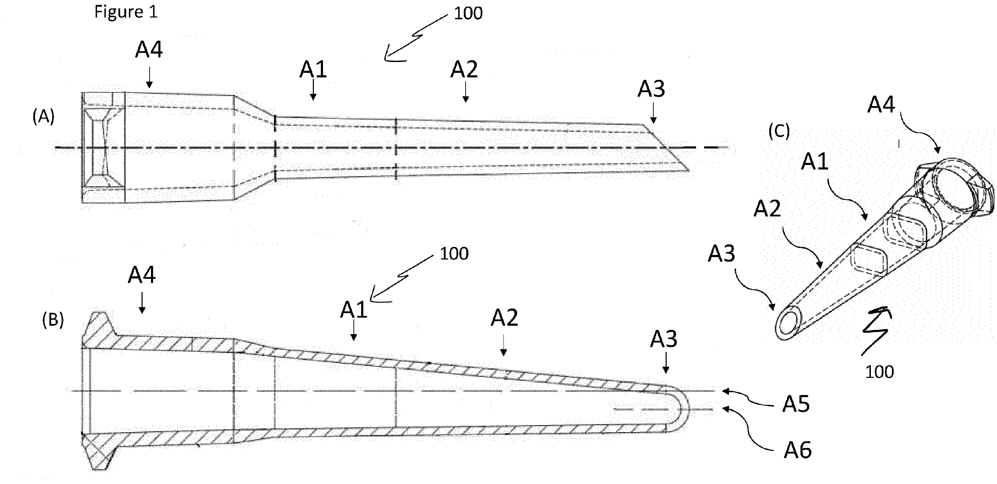

**Figure 1 (A) to (C)** illustrate one embodiment of the injector 100 according to the invention. Figure (A) and (B) show a cross section of the injector 100, while Figure 1(C) shows a perspective view. The injector 100 comprises a first opening A4, a first compartment A1, a second compartment A2 and a second opening A3. In the cross section shown in Figure 1(B) it can be seen that the central axis of the second compartment A6 is shifted from the central axis of the first compartment A5. This embodiment is characterized by the following features:
∘ Shape: Squared rectangular or slightly rounded
∘ Purpose: Short-term storage, transportation and transplantation
∘ Suitable for: Very thin DSAEK grafts under 50 microns
∘ Recommended usage: Cases requiring minimal preservation time before transplantation

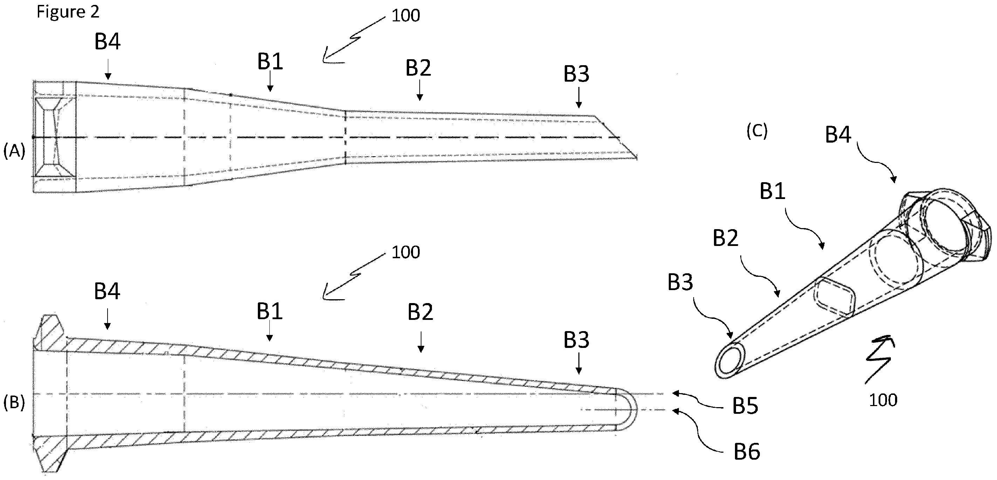

**Figure 2 (A) to (C)** illustrate one embodiment of the injector 100 according to the invention. Figure (A) and (B) show a cross section of the injector 100, while Figure 2(C) shows a perspective view. The injector 100 comprises a first opening B4, a first compartment B1, a second compartment B2 and a second opening B3. In the cross section shown in Figure 2(B) it can be seen that the central axis of the second compartment B6 is shifted from the central axis of the first compartment B5. This embodiment is characterized by the following features:
∘ Shape: Fully rounded or oval
∘ Purpose: Common storage and transportation for a few days and transplantation
∘ Suitable for: DSAEK grafts under 100 microns
∘ Recommended usage: Standard storage and transport situations

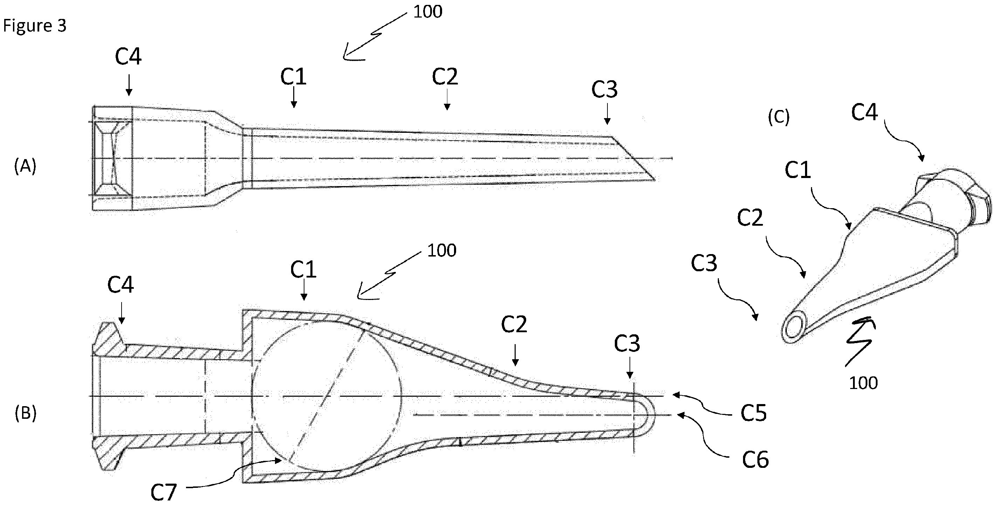

**Figure 3 (A) to (C)** illustrate one embodiment of the injector 100 according to the invention. Figure (A) and (B) show a cross section of the injector 100, while Figure 3(C) shows a perspective view. The injector 100 comprises a first opening C4, a first compartment C1, a second compartment C2 and a second opening C3. In the cross section shown in Figure 3(B) it can be seen that the central axis of the second compartment C6 is shifted from the central axis of the first compartment C5. The inner hollow part of the first compartment C1 offers space for a DSAEK tissue graft in natural state. Therefore, the first compartment provides a space with the diameter C7. This embodiment is characterized by the following features:
∘ Shape: Rectangular or square with rounded corners
∘ Purpose: Long-term storage, transportation and transplantation
∘ Suitable for: any DSAEK grafts and even Full-stroma thickness grafts
∘ Recommended usage: Extended preservation while maintaining the native conformation of the Graft

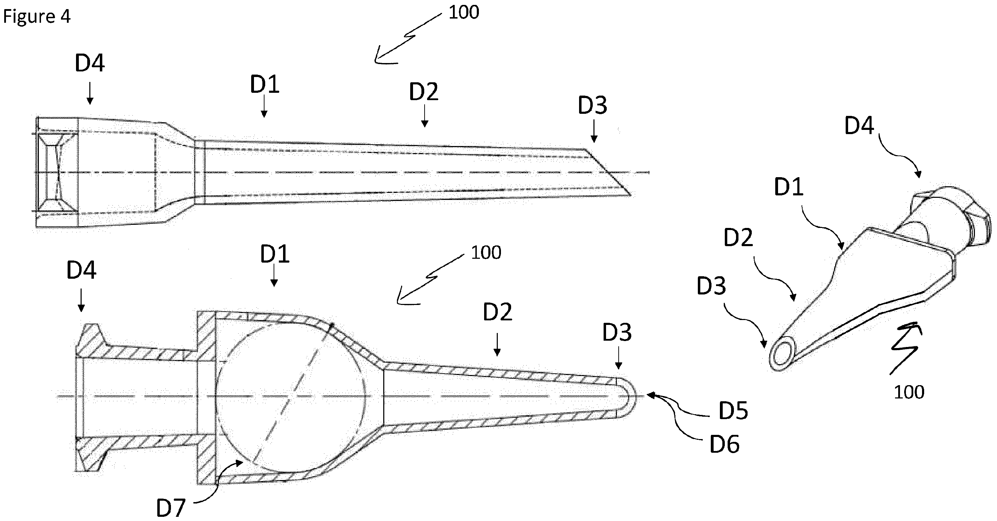

**Figure 4 (A) to (C)** illustrate one embodiment of the injector 100 according to the invention. Figure (A) and (B) show a cross section of the injector 100, while Figure 4(C) shows a perspective view. The injector 100 comprises a first opening D4, a first compartment D1, a second compartment D2 and a second opening D3. In the cross section shown in Figure 4(B) it can be seen that the central axis of the second compartment D6 is not shifted from the central axis of the first compartment D5. This embodiment is characterized by the following features:
∘ Shape: Rectangular or square with rounded corners
∘ Purpose: Long-term storage, transportation and transplantation
∘ Suitable for: any DSAEK grafts and even Full-stroma thickness grafts
∘ Recommended usage: Extended preservation while maintaining the native conformation of the Graft

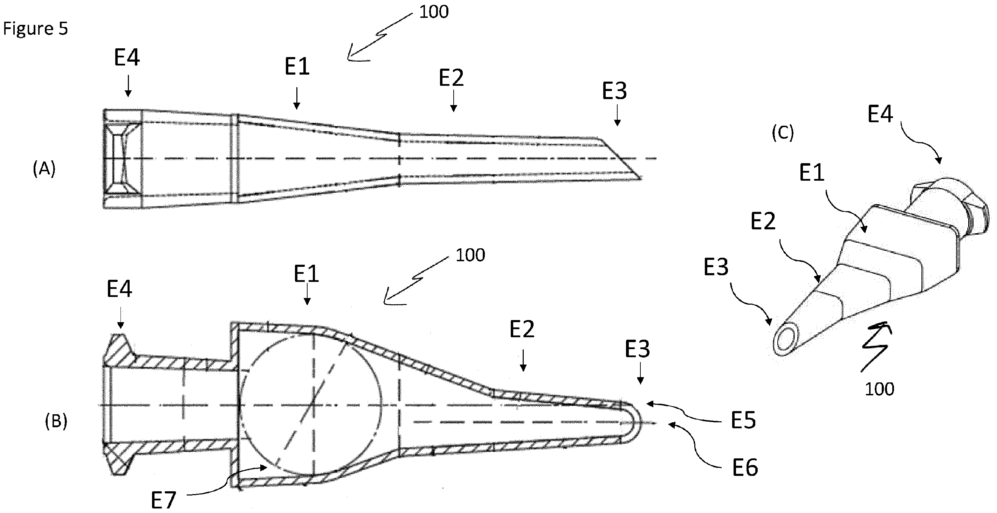

**Figure 5 (A) to (C)** illustrate one embodiment of the injector 100 according to the invention. Figure (A) and (B) show a cross section of the injector 100, while Figure 5(C) shows a perspective view. The injector 100 comprises a first opening E4, a first compartment E1, a second compartment E2 and a second opening E3. In the cross section shown in Figure 5(A) it can be seen that a first compartment E1 has a large volume in compare to C1 in Figure 3. In the cross section shown in Figure 5(B) it can be seen that the central axis of the second compartment E6 is shifted from the central axis of the first compartment E5. The inner hollow part of the first compartment E1 offers space for a DSAEK tissue graft in natural state. Therefore, the first compartment provides a space with the diameter E7. This embodiment is characterized by the following features:
∘ Shape: Rectangular or square with rounded corners
∘ Purpose: Long-term storage, transportation and transplantation
∘ Suitable for: any DSAEK grafts and even Full-stroma thickness grafts
∘ Recommended usage: Extended preservation while maintaining the native conformation of the Graft

## Claims

1. Device for Descement Stripping Automated Endothelial Keratoplasty (DSAEK) comprising a main body, wherein the main body comprises
• a first compartment as transportation and storage section comprising a central axis in longitudinal extension of the main body;
• a second compartment as insertion section comprising a central axis in longitudinal direction of the main body; and
• a first and a second opening;
wherein the central axis of the first compartment does not coincide with the central axis of the second compartment.

2. Device according to claim 1, **characterized in that** the central axis of the first compartment is shifted in parallel to the central axis of the second compartment.

3. Device according to claim 2, **characterized in that** the central axis of the second compartment is shifted from the central axis of the first compartment between 1/3 to 1/2 of the opening diameter of the second opening.

4. Device according to any of the preceding claims, **characterized in that** the second compartment comprises two opposing angled surfaces, wherein each surface is angled between 0° and 10°.

5. Device according to any of the preceding claims, **characterized in that** the first compartment has a round, rectangular or squared shaped part.

6. Device according to any of the preceding claims, **characterized in that** first opening comprises a luer connector.

7. Device according to any of the preceding claims, **characterized in that** the second opening is located on the second compartment and has a diameter between 1.0 mm and 1.8 mm.

8. Device according to any of the preceding claims, **characterized in that** the device further comprises a cap or a lid for the first opening and/or for the second opening.

9. Device according to any of the preceding claims, **characterized in that** first compartment and/or second compartment is transparent.

10. Device according to any of the preceding claims, **characterized in that** the injector consists of glass or plastic.

11. Device according to any of the preceding claims, **characterized in that** second compartment comprises a grid.

12. Device according to any of the preceding claims, **characterized in that** the inner surface of the injector comprises a hydrophobic coating.

13. A method for implanting a tissue graft for DSAEK using the device according to any of claims 1 to 12 comprising the steps
• loading a DSAEK tissue graft into the first compartment;
• arranging a macroporous material in the second opening;
• positioning the tissue graft within the second compartment using a liquid flow;
• remove the macroporous material from the second opening.
• eject the tissue graft from the injector into the anterior chamber of an eye.

14. A method for transporting a tissue graft for DSAEK using the device according to any of claims 1 to 12 comprising the steps
• Filling the device with a medium
• loading a DSAEK tissue graft into the first compartment;
• closing the first opening with a cap preferably luer lock lid;
• positioning the device into a container with medium, for storage and transportation.

15. A method for transporting a tissue graft for DSAEK using the device according to any of claims 1 to 12 comprising the steps
• Filling the device with medium
• loading a DSAEK tissue graft into the first compartment;
• closing the large opening with a cap preferably luer lock lid;
• closing the second opening with a cap
• positioning the device into a container, for storage and transportation.

## Amended claims

### Amended claims in accordance with Rule 137(2) EPC.

1. Device (100) for Descement Stripping Automated Endothelial Keratoplasty (DSAEK) comprising a main body, wherein the main body comprises
• a first compartment (A1, B1, C1, D1, E1) as transportation and storage section comprising a central axis (A5, B5, C5, D5, E5) in longitudinal extension of the main body;
• a second compartment (A2, B2, C2, D2, E2) as insertion section comprising a central axis (A6, B6, C6, D6, E6) in longitudinal direction of the main body; and
• a first (A4, B4, C4, D4, E4) and a second opening (A3, B3, C3, D3, E3);
**characterized in that**,
the central axis of the first compartment (A5, B5, C5, D5, E5) does not coincide with the central axis of the second compartment (A6, B6, C6, D6, E6).

2. Device (100) according to claim 1, **characterized in that** the central axis of the first compartment (A5, B5, C5, D5, E5) is shifted in parallel to the central axis of the second compartment (A6, B6, C6, D6, E6).

3. Device (100) according to claim 2, **characterized in that** the central axis of the second compartment (A6, B6, C6, D6, E6) is shifted from the central axis of the first compartment (A5, B5, C5, D5, E5) between 1/3 to 1/2 of the opening diameter of the second opening.

4. Device (100) according to any of the preceding claims, **characterized in that** the second compartment (A2, B2, C2, D2, E2) comprises two opposing angled surfaces, wherein each surface is angled between 0° and 10°.

5. Device (100) according to any of the preceding claims, **characterized in that** the first compartment (A1, B1, C1, D1, E1) has a round, rectangular or squared shaped part.

6. Device (100) according to any of the preceding claims, **characterized in that** first opening (A4, B4, C4, D4, E4) comprises a luer connector.

7. Device (100) according to any of the preceding claims, **characterized in that** the second opening (A3, B3, C3, D3, E3) is located on the second compartment (A2, B2, C2, D2, E2) and has a diameter between 1.0 mm and 1.8 mm.

8. Device (100) according to any of the preceding claims, **characterized in that** the device further comprises a cap or a lid for the first opening (A4, B4, C4, D4, E4) and/or for the second opening (A3, B3, C3, D3, E3).

9. Device (100) according to any of the preceding claims, **characterized in that** first compartment (A1, B1, C1, D1, E1) and/or second compartment (A2, B2, C2, D2, E2) is transparent.

10. Device (100) according to any of the preceding claims, **characterized in that** the injector (100) consists of glass or plastic.

11. Device (100) according to any of the preceding claims, **characterized in that** second compartment (A2, B2, C2, D2, E2) comprises a grid.

12. Device (100) according to any of the preceding claims, **characterized in that** the inner surface of the injector (100) comprises a hydrophobic coating.

13. A method for preparing a tissue graft for DSAEK using the device (100) according to any of claims 1 to 12 comprising the steps
• loading a DSAEK tissue graft into the first compartment (A1, B1, C1, D1, E1);
• arranging a macroporous material in the second opening (A3, B3, C3, D3, E3);
• positioning the tissue graft within the second compartment (A2, B2, C2, D2, E2) using a liquid flow.

14. A method for transporting a tissue graft for DSAEK using the device according to any of claims 1 to 12 comprising the steps
• filling the device (100) with a medium;
• loading a DSAEK tissue graft into the first compartment (A1, B1, C1, D1, E1);
• closing the first opening (A4, B4, C4, D4, E4) with a cap preferably a luer lock lid;
• positioning the device (100) into a container with medium, for storage and transportation.

15. A method for transporting a tissue graft for DSAEK using the device according to any of claims 1 to 12 comprising the steps
• filling the device (100) with medium;
• loading a DSAEK tissue graft into the first compartment (A1, B1, C1, D1, E1);
• closing the large opening with a cap preferably luer lock lid;
• closing the second opening (A3, B3, C3, D3, E3) with a cap;
• positioning the device (100) into a container, for storage and transportation.
